# EUROPEAN PATENT APPLICATION

(11) **EP 4 382 025 A1**
(43) Date of publication of application: **12.06.2024**
(21) Application number: 22853240.4
(22) Date of filing: 20.05.2022
(51) Int. Cl.: A61B 1/06, A61B 1/07, A61B 1/00, A61B 1/05, G02B 23/24

(54) **MULTISPECTRUM ENDOSCOPE AND ENDOSCOPE SYSTEM COMPRISING SAME**

(30) Priority: 06.08.2021 KR 20210103771
(71) Applicant: Metaplebio Co., Ltd., Seoul 06677 (KR)
(72) Inventor: HAN, Young Geun, Seoul 05834 (KR); PARK, Hong Seong, Seoul 02560 (KR)
(74) Representative: Sander, Rolf
(86) International application number: PCT/KR2022/007234
(87) International publication number: WO 2023/013860

(57) **Abstract**

The present invention relates to a multispectrum endoscope and an endoscope system comprising same. The multispectrum endoscope of the present invention comprises: a first light source emitting infrared rays having a plurality of wavelengths; a plurality of multimode optical fibers for guiding the infrared rays emitted from the first light source toward a sample; a second light source emitting a visible ray; an optical fiber for guiding the visible ray emitted from the second light source toward the sample; a lens part for receiving a fluorescent signal emitted from the sample and a visible light signal reflected off the sample; and an optical fiber bundle for guiding the fluorescent signal and/or the visible light signal received by the lens part toward a multispectrum photodetector, wherein the optical fiber bundle is disposed so as to be surrounded by the plurality of multimode optical fibers, and the end portion of the multispectrum endoscope emitting the visible ray surrounds at least a portion of the plurality of multimode optical fibers.

## Description

### Technical Field

The disclosure relates to a multi-spectrum endoscope and an endoscope system including the same, and more particularly, to a multi-spectrum endoscope capable of reducing the measurement, identification, and noise of a plurality of fluorescent signals according to a laser that is radiated through a plurality of wavelengths and an endoscope system including the same.

### Background Art

An endoscope is inserted deep into the inside of the human body along an organ after entering the human body due to its flexible characteristic. A doctor may check and diagnose the inside of the human body while seeing an image or video that is photographed through a lens of the endoscope.

As the endoscope photographs the inside of the human body while moving within the human body as described above, the endoscope needs to be very slimly constructed so that a person rarely feels inconvenient. If a plurality of light sources is used in the endoscope, there is a problem in that a person may feel inconvenient because a total thickness of the endoscope is inevitably increased. Accordingly, there are technical limitations in developing an endoscope using a plurality of light sources

Furthermore, the endoscope also includes a light source and a lens capable of performing image processing on a light signal that is transmitted from the light source to a cell and that is transmitted to the endoscope again. That is, if the light source is used as an infrared laser in the endoscope, a common fluorescent imaging system that detects a fluorescent signal that is emitted after the infrared laser is radiated to the cell may be applied. Such a common fluorescent imaging system concentrates exciting light having one wavelength in clinical trials, animal testing, pattern-guided surgery, etc., passes the exciting light through an object lens, and radiates the exciting light to a sample, that is, a monitoring portion, so that a change or structure of the sample according to a light signal that is reflected or discharged from the sample can be monitored.

As exciting light having a single wavelength is used in a conventional fluorescent imaging system as described above, it is impossible to excite a light-emitting body by using various wavelengths. Furthermore, it is impossible to simultaneously observe multiple fluorescent images having multiple spectra because the wavelength of exciting light is not varied in the light source.

Furthermore, when exciting light having one wavelength is emitted from the light source, it is impossible to adjust the area of a field of view in a single light source or between a plurality of overlapped light sources. Furthermore, if a laser light source is used, the quality of an obtained fluorescent image may be degraded because speckle noise attributable to an interference phenomenon between pieces of scattered light and multi-modal noise attributable to multiple modes occurs.

In this case, the "speckle noise" means noise that appears in the form of a speckled pattern because a laser light source is radiated to a sample or die and a glare and a shadow are irregularly distributed due to a random interference phenomenon of light that is scattered from the sample or die if a laser is used as the light source. That is, in the speckle noise, bright or dark points are scattered and appear in a photographed image due to interference that occurs because the laser is scattered in various directions. Furthermore, the multi-modal noise is multi-modal pattern noise that occurs due to multiple modes that are formed as a laser passes through a multi-mode optical fiber, and may degrade the quality of a fluorescent image.

If a laser is used as a light source as described above, there are problems in that the quality of a photographed fluorescent image is degraded due to speckle noise and multi-modal noise and thus costs and time, such as that additional photographing is required because the accuracy of image reading is reduced, are increased.

Meanwhile, the aforementioned background technology is technical information that has been owned by an inventor in order to derive the present disclosure or that is obtained in a process of deriving the present disclosure, and may not be said to be essentially a known technology that has been disclosed to the common public prior to the application of the present disclosure.

(Prior Art Document) Korean Patent Application Publication No. 10-2006-0108713 (October 18, 2006)

### DISCLOSURE

### Technical Problem

An object to be solved by the present disclosure is to provide a multi-spectrum endoscope capable of identifying, distinguishing, and outputting multi-wavelength fluorescent signals that emit different wavelengths by different contrast media by radiating pieces of laser light having a plurality of wavelengths to a sample or a cell in a small endoscope, and an endoscope system including the same.

Furthermore, another object to be solved by the present disclosure is to provide a multi-spectrum endoscope capable of obtaining and distinguishing fluorescent signals having different wavelengths through a wavelength-variable light source, a multi-wavelength light source, a multi-array light source, etc., and an endoscope system including the same.

Furthermore, still another object to be solved by the present disclosure is to provide a multi-spectrum endoscope capable of reducing speckle noise and multi-modal noise attributable to pieces of laser light having a plurality of wavelengths, and an endoscope system including the same.

Furthermore, still another object to be solved by the present disclosure is to provide a multi-spectrum endoscope capable of simultaneously outputting a visible light signal and fluorescent signals having different wavelengths by separating the visible light signal and the fluorescent signals through a light separator, and an endoscope system including the same.

Furthermore, still another object to be solved by the present disclosure is to provide a multi-spectrum endoscope capable of detecting fluorescent signals having different wavelengths by emitting a laser having a specific wavelength range for the necrosis or treatment of a specific cell and simultaneously treating an affected area by directly radiating a laser to the affected area, and an endoscope system including the same.

Technical objects of the present disclosure are not limited to the aforementioned objects, and the other objects not described above may be evidently understood from the following description by those skilled in the art.

### Technical Solution

In order to solve the aforementioned object, a multi-spectrum endoscope according to an embodiment of the present disclosure includes a first light source configured to emit an infrared ray having a plurality of wavelengths, a plurality of multi-mode optical fibers configured to provide an infrared ray that is emitted from the first light source with guidance toward a sample, a second light source configured to emit visible light, an optical fiber configured to provide the infrared ray that is emitted from the second light source with guidance toward the sample, a lens part configured to receive a fluorescent signal that is emitted from the sample and a visible light signal that is reflected by the sample, and an optical fiber bundle configured to provide at least one of the fluorescent signal and the visible light signal that are received through the lens part with guidance toward a multi-spectrum light detection device. The optical fiber bundle is disposed to be surrounded by the plurality of multi-mode optical fibers. An end part of the multi-spectrum endoscope from which the visible light is emitted surrounds at least some of the plurality of multi-mode optical fibers.

A multi-spectrum endoscope according to another characteristic of the present disclosure may further include a coupler configured to separate the infrared ray having the plurality of wavelengths into individual infrared rays having different wavelengths or having pieces of different predetermined power.

According to still another characteristic of the present disclosure, the coupler may transmit at least one of the fluorescent signal and the visible light signal toward the light detection device.

According to still another characteristic of the present disclosure, each of the plurality of multi-mode optical fibers may output the individual infrared ray separated by the coupler by providing the individual infrared ray with guidance toward the sample.

According to still another characteristic of the present disclosure, the first light source may be a wavelength-variable light source, a multi-wavelength light source, or a multi-array light source, and may emit light having a different wavelength over time.

According to still another characteristic of the present disclosure, at least one of the plurality of multi-mode optical fibers may output a beam between 200 nm to 450 nm, a beam between 510 nm to 550 nm, a near-infrared ray between 790 nm to 980 nm, or a near-infrared ray between 1044 nm to 1084 nm.

According to still another characteristic of the present disclosure, an end part of each of the plurality of multi-mode optical fibers from which the first light source is emitted may include a homogenizer or a diffuser.

In order to solve the aforementioned object, a multi-spectrum endoscope system according to an embodiment of the present disclosure includes a multi-spectrum endoscope according to an embodiment of the present disclosure, a multi-spectrum light detection device configured to detect at least one of a fluorescent signal that is radiated from the multi-spectrum endoscope to a sample and that is then emitted from the sample and a visible light signal that is reflected by the sample, and a control device configured to synchronize the multi-spectrum endoscope and the multi-spectrum light detection device.

According to another characteristic of the present disclosure, the multi-spectrum light detection device may include a wavelength separator configured to separate the fluorescent signal and the visible light signal, a band rejection filter disposed between the sample and the wavelength separator, an infrared image detection unit configured to detect the fluorescent signal, and a visible light image detection unit configured to detect the visible light signal.

According to still another characteristic of the present disclosure, the infrared image detection unit may include an infrared image sensor, an infrared object lens, and a wheel filter. The visible light image detection unit may include a visible light image sensor and a visible light object lens. The wheel filter may be disposed between the wavelength separator and the infrared image sensor or between the wavelength separator and the band rejection filter.

According to still another characteristic of the present disclosure, the control device may synchronize a time between the first light source and the infrared image sensor and a wavelength of light corresponding to the time, may synchronize a time between the first light source and the wheel filter and a wavelength of light corresponding to the time, and may synchronize a time between the wheel filter and the infrared image sensor and a wavelength of light corresponding to the time.

### Advantageous Effects

According to any one of the solving means of the present disclosure, in an embodiment of the present disclosure, fluorescent images having different wavelengths can be obtained and identified.

Furthermore, according to any one of the solving means of the present disclosure, in another embodiment of the present disclosure, speckle noise and multi-modal noise attributable to a laser can be reduced.

Furthermore, according to any one of the solving means of the present disclosure, in still another embodiment of the present disclosure, a visible light signal and fluorescent signals having different wavelengths can be separated and simultaneously output.

Furthermore, according to any one of the solving means of the present disclosure, in still another embodiment of the present disclosure, fluorescent signals having different wavelengths can be detected, and simultaneously an affected area can be treated by directly radiating a laser to the affected area.

Effects of the present disclosure which may be obtained in the present disclosure are not limited to the aforementioned effects, and the other effects not described above may be evidently understood by a person having ordinary knowledge in the art to which the present disclosure pertains from the following description.

### Brief Description of Drawings

FIG. 1 schematically illustrates an endoscope system including a multi-spectrum endoscope according to an embodiment of the present disclosure.
FIG. 2 is a cross-sectional view of the end part of the multi-spectrum endoscope toward a sample according to an embodiment of the present disclosure.
FIG. 3 is a cross-sectional view of the multi-spectrum endoscope according to an embodiment of the present disclosure, which is taken along in a direction III-III' in FIG. 1.
FIG. 4 is a schematic block diagram of a multi-spectrum light detection device that is connected to the multi-spectrum endoscope according to an embodiment of the present disclosure.
FIG. 5 is a block diagram for describing a method of identifying and measuring a multi-wavelength fluorescent signal by synchronizing the multi-spectrum endoscope and the multi-spectrum light detection device in the endoscope system according to an embodiment of the present disclosure.

### Mode for Invention

Advantages and characteristics of the present disclosure and a method for achieving the advantages and characteristics will become apparent from embodiments described in detail later in conjunction with the accompanying drawings. However, the present disclosure is not limited to the disclosed embodiments, but may be implemented in various different forms. The embodiments are merely provided to complete the present disclosure and to fully notify a person having ordinary knowledge in the art to which the present disclosure pertains of the category of the present disclosure. The present disclosure is merely defined by the category of the claims.

A shape, a size, a ratio, an angle, a number, etc. disclosed in the drawings for describing the embodiments of the present disclosure are illustrative, and the present disclosure is not limited to illustrated contents. Furthermore, in describing the present disclosure, a detailed description of a related known technology will be omitted if it is deemed to make the subject matter of the present disclosure unnecessarily vague. If a term, such as "include", "have" or "consist of" mentioned in this specification, is used, another part may be added unless "only ~" is used. If a component is expressed in the singular form, it includes a case in which the element is a plural form unless specially described otherwise.

In interpreting a component, the interpretation is construed as including an error range unless explicitly described otherwise separately.

A first, a second, etc. are used to describe various components, but the components are not restricted by the terms. The terms are used to only distinguish one component from the other components. Accordingly, a first component that is described hereinafter may be a second component within the technical spirit of the present disclosure.

Throughout the specification, the same reference numeral denotes the same component unless separately specified.

Characteristics of several embodiments of the present disclosure may be partially or entirely coupled or combined and may be technically variously associated and driven as may be sufficiently understood by those skilled in the art. The embodiments may be independently implemented and may be implemented in an associative relation.

Meanwhile, a potential effect that has not been specifically mentioned in the specification of the present disclosure and that may be expected by technical characteristics of the present disclosure is treated as if it has been described in this specification. The present embodiment has been provided to a person having ordinary knowledge in the art to more fully describe the present disclosure. Contents illustrated in the drawings may be exaggerated and represented compared to an implementation form of an actual invention. A detailed description of a component will be omitted or described in brief if it is deemed to make the subject matter of the present disclosure unnecessarily vague.

In this specification, light having a plurality of wavelengths may be light consisting of one beam simultaneously having a plurality of different wavelengths, may be light consisting of a separate beam for each wavelength and beams having a plurality of different wavelengths, and may mean wavelength-variable light over time in one beam. Accordingly, a light source that emits light having a plurality of wavelengths may be a multi-wavelength light source, a multi-array light source, a wavelength-variable light source, etc. Each of such light sources may emit light having different wavelengths simultaneously and at different times.

In this specification, a control device is a device capable of controlling the components of a multi-spectrum endoscope system of the present disclosure. The control device is connected to a multi-spectrum endoscope and light detection device of the present disclosure in a wired or wireless way so that the control device may electrically communicate with the multi-spectrum endoscope and the light detection device, and may generate a signal that controls the multi-spectrum endoscope and the light detection device and supply the signal thereto. Such a control device includes a processor and memory, and may include various electronic devices including a computer, a mobile device, and an embedded program, for example. Furthermore, the control device may further include an image processing processor that processes and outputs an image by receiving a light signal through the light detection device according to an embodiment of the present disclosure. Furthermore, the control device may enable an image that has been processed through the image processing processor to be output through a display device connected thereto. Various examples of the control device are not limited to the aforementioned contents, and include all devices capable of generating and transmitting a signal for controlling the multi-spectrum endoscope system and processing data that have been received from a fluorescent image system.

Hereinafter, embodiments of the present disclosure are described in detail with reference to the accompanying drawings so that a person having ordinary knowledge in the art to which the present disclosure pertains may easily practice the embodiments. The present disclosure may be implemented in various different forms and is not limited to the embodiments described herein. In the drawings, in order to clarify a description of the present disclosure, parts not related to the description are omitted, and the same reference numbers are used to refer to the same or similar parts throughout the specification. Furthermore, the size and thickness of each of components shown in the drawings are arbitrarily illustrated for convenience of description, and thus the present disclosure is not essentially limited thereto.

Hereinafter, the present disclosure is described in detail with reference to the accompanying drawings.

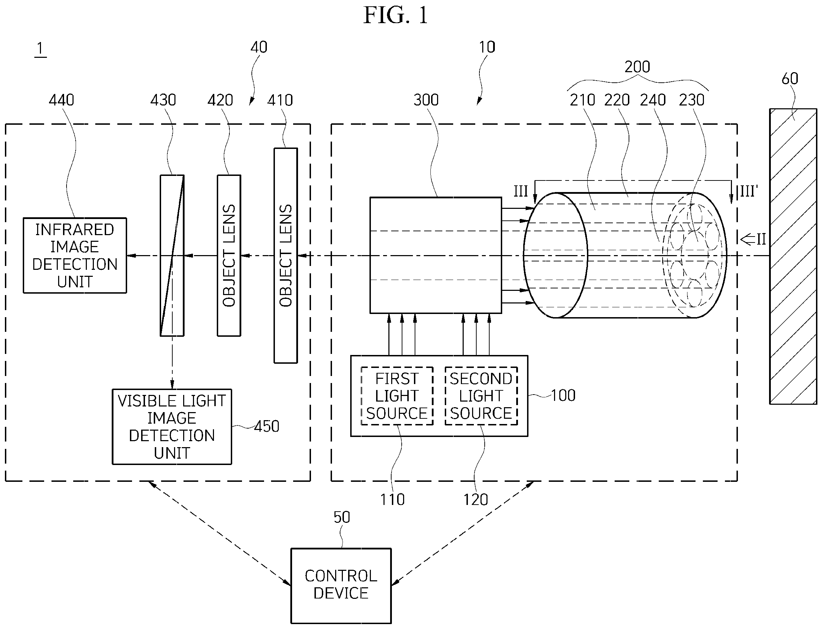

FIG. 1 schematically illustrates an endoscope system including a multi-spectrum endoscope according to an embodiment of the present disclosure.

Referring to FIG. 1, a multi-spectrum endoscope system 1 according to an embodiment of the present disclosure includes a multi-spectrum endoscope 10 (hereinafter referred to as an "endoscope"), a multi-spectrum light detection device 40 (hereinafter referred to as a "light detection device"), and a control device 50.

Specifically, the endoscope 10 includes a light source 100, an insertion unit 200, and a coupler 300. The light source 100 includes a first light source 110 and a second light source 120. The insertion unit 200 includes a plurality of multi-mode optical fibers 210, at least one optical fiber or lighting part 220, a lens part 230, and an optical fiber bundle 240.

Referring to FIG. 1, infrared rays having a plurality of wavelengths, which are emitted from the light source 100, and visible light are transferred to the insertion unit 200 through the coupler 300. Specifically, the infrared rays and the visible light that are emitted from the light source 100 are separated into individual infrared rays and visible light having different wavelengths or different power in the coupler 300. The individual infrared rays may be transferred to a sample 60 through the plurality of multi-mode optical fibers 210. The visible light may be transferred to the sample 60 through the at least one optical fiber or lighting part 220. That is, the infrared rays that are emitted from the first light source 110 and the visible light that is emitted from the second light source 120 may be transferred to the insertion unit 200 through the coupler 300.

Furthermore, referring to FIG. 1, the individual infrared rays and the visible light are radiated to the sample 60. A fluorescent signal that is emitted from the sample 60 and a visible light signal that is reflected by the sample 60 are directed toward the lens part 230. That is, the lens part 230 receives the fluorescent signal that is emitted from the sample 60 and the visible light signal that is reflected by the sample 60. The fluorescent signal and the visible light signal that have been received through the lens part 230 as described above are transferred to the light detection device 40 through the optical fiber bundle 240.

The light source 100 is a component that emits light having a plurality of wavelengths. Preferably, the light source 100 may emit various types of light.

The first light source 110 is a light source that emits an infrared ray having a plurality of wavelengths. Specifically, the first light source 110 may be constructed to emit an infrared ray having different wavelengths over time. The first light source 110 may be any one of a wavelength-variable light source, a multi-wavelength light source, or a multi-array light source. Furthermore, the first light source may be constructed to emit an infrared ray having pieces of different power over time.

The first light source 110 may emit infrared rays having a plurality of different wavelengths to different multi-mode optical fiber 210, respectively, through the coupler 300 at different times (a multi-wavelength light source), may emit an infrared ray having a desired wavelength to one multi-mode optical fiber 210 via the coupler 300 at a different time (a wavelength-variable light source), and may simultaneously emit infrared rays having a plurality of different wavelengths to different multi-mode optical fibers 210, respectively, via the coupler 300 (a multi-array light source). For example, if the first light source 110 is the multi-wavelength light source, the first light source 110 may emit a laser having a specific wavelength λ1 at an arbitrary time t1 through one multi-mode optical fiber 210a, may emit a laser having another specific wavelength λ2 at another time t2 through another multi-mode optical fiber 210b, and may emit a laser having still another specific wavelength λ3 at still another time t3 through still another multi-mode optical fiber 210c. Furthermore, if the light source 110 is the wavelength-variable light source, the light source 110 may emit a laser having a specific wavelength λ1 from an arbitrary time t1 to another time t2 through the multi-mode optical fibers 210, may emit a laser having another specific wavelength λ2 from the another time t2 to still another time t3, and may emit a laser having still another specific wavelength λ3 from the still another time t3 to still another time t4.

The infrared ray that is emitted from the first light source 110 may be preferably a near-infrared ray. More preferably, the infrared ray that is emitted from the first light source 110 may be a near-infrared ray. For example, the wavelength of the near-infrared ray that is emitted from the first light source 110 may be about 200 nm to about 3000 nm. The first light source 110 may emit a beam close to the wavelength region of the near-infrared ray. For example, the first light source 110 may also emit a beam between about 200 nm to about 550 nm. The first light source 110 may emit light having different wavelengths over time. As described above, the first light source 110 that emits an infrared ray having various wavelengths or power may be a laser using an LED, an LD, or a semiconductor, an optical fiber laser, a quantum dot laser, or semiconductor optical amplifier light source, for example. Furthermore, the first light source 110 may emit a laser for treatment purposes. The laser for treatment purposes may emit a beam between 200 nm to 450 nm, a beam between 510 nm to 550 nm, a near-infrared ray between 790 nm to 980 nm, or a near-infrared ray between 1044 nm to 1084 nm. In particular, a near-infrared ray having 808 nm, among lasers or beams that are emitted through the first light source 110, may remove or treat a cancer cell on the sample 60. Pieces of light that are emitted through the first light source 110 as described above may simultaneously remove or treat a cancer cell by being radiated to the sample 60 through various wavelengths so that a fluorescent signal is emitted.

The second light source 120 is a light source that is directed toward the sample 60, and may be illumination light that emits visible light. In this case, the illumination light is light that plays a role as lighting that brightly shines the sample, and may include bright light having visible light wavelength region. In particular, in some embodiments of the present disclosure, the illumination light may be white light. Preferably, lighting that emits the illumination light may be implemented as a white LED.

The plurality of multi-mode optical fibers 210 of the insertion unit 200 provides an infrared ray that is emitted from the first light source 110 with guidance toward the sample 60. Specifically, each of the plurality of multi-mode optical fibers 210 may provide individual infrared rays having different wavelengths, which have been separated by the coupler 300, or individual infrared rays having pieces of different power with guidance toward the sample 60, and may emit the individual infrared rays toward the sample 60.

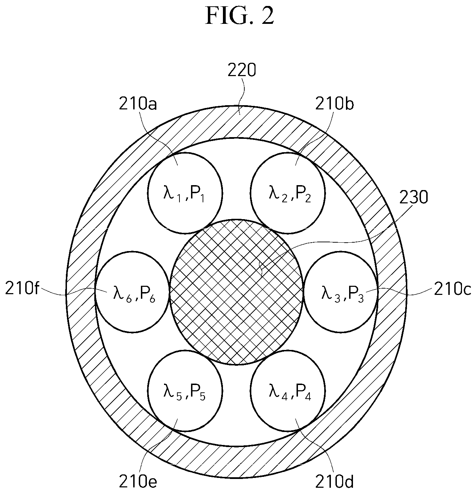

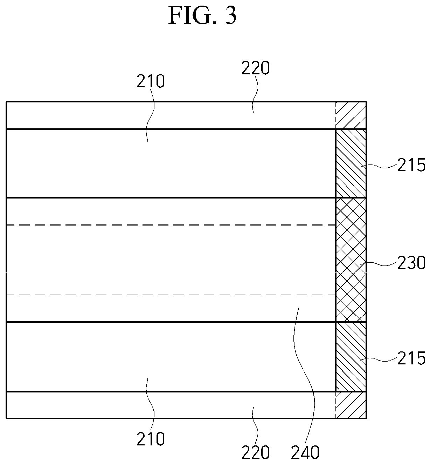

The at least one optical fiber or lighting part 220 of the insertion unit 200 provides visible light that is emitted from the second light source 120 with guidance toward the sample 60. The optical fiber or lighting part 220 may output the visible light toward the sample 60 by providing the visible light with guidance. In particular, the optical fiber or lighting part 220 is connected to an end part of the endoscope 10, and outputs visible light. The visible light that is transferred to the end part of the endoscope 10 through the optical fiber or lighting part 220 may be emitted through an opening of the end part of the endoscope 10 that surrounds the plurality of multi-mode optical fibers 210. Detailed structures of the plurality of multi-mode optical fibers 210, the optical fiber or lighting part 220, and the opening of the end part of the endoscope 100, which may be monitored in a cross section of the insertion unit 200, are described later with reference to FIGS. 2 and 3.

In the insertion unit 200, the lens part 230 receives a fluorescent signal that is emitted from the sample 60 and a visible light signal that is reflected by the sample 60. The lens part 230 is an object lens toward the sample 60, and may be composed of a microlens. The lens part 230 is connected to the optical fiber bundle 240. Light signals that are received by the lens part 230 may be transferred toward the light detection device 40 through the optical fiber bundle 240.

In the insertion unit 200, the optical fiber bundle 240 provides at least one of the fluorescent signal and the visible light signal that are received through the lens part 230 with guidance toward the light detection device 40. Such an optical fiber bundle 240 is disposed to be surrounded by the plurality of multi-mode optical fibers 210 as illustrated in FIG. 1.

The coupler 300 separates an infrared ray having a plurality of wavelengths into individual infrared rays having different wavelengths or predetermined individual power. Furthermore, each of the individual infrared rays separated by the coupler 300 is transferred to the insertion unit 200. Specifically, each of the individual infrared rays separated by the coupler 300 is transferred to the sample 60 through the plurality of multi-mode optical fibers 210 of the insertion unit 200.

The coupler 300 may separate an infrared ray having a plurality of wavelengths, which is emitted from the first light source 110, into a plurality of individual infrared rays corresponding to a plurality of wavelengths, respectively, or may separate the infrared ray into a plurality of individual infrared rays corresponding to a plurality of pieces of powers, respectively, on the basis of a plurality of pieces of predetermined power. For example, the coupler 300 may emit infrared rays having a plurality of different wavelengths at different times by separating the infrared rays into different multi-mode optical fibers 210 (connected to the multi-wavelength light source), may emit an infrared ray having a desired wavelength to one multi-mode optical fiber 210 at a different time (connected to the wavelength-variable light source), and may simultaneously emit infrared rays having a plurality of different wavelengths to the different multi-mode optical fibers 210, respectively (connected to the multi-array light source).

Furthermore, according to some other embodiments, the coupler 30 may transmit at least one of fluorescent signals and a visible light signal that are received through the lens part 230 so that the at least one signal is directed toward the light detection device 40.

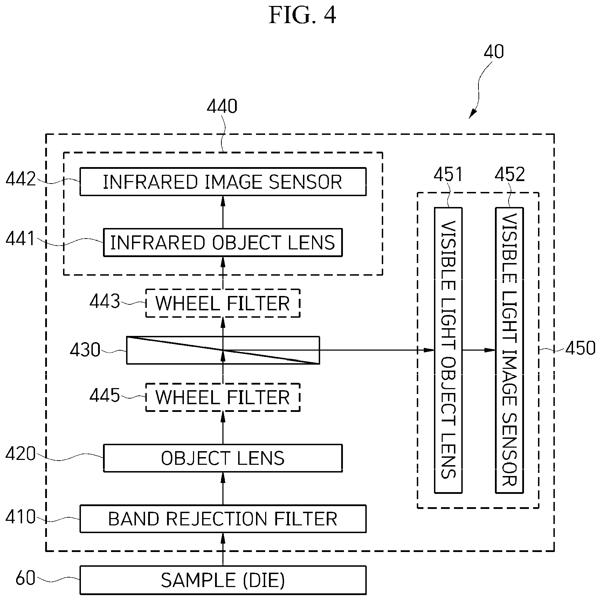

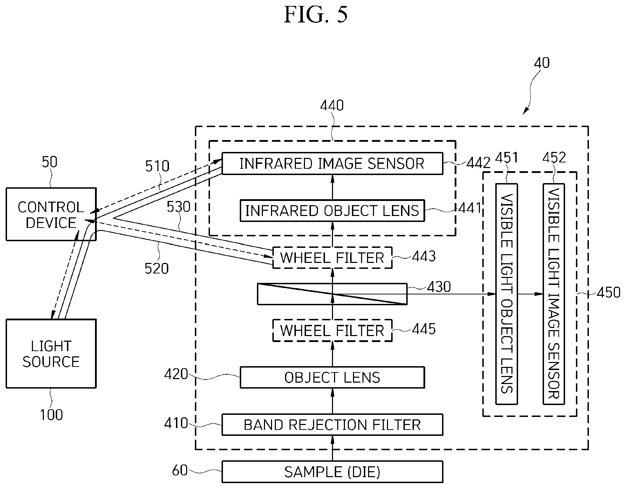

The light detection device 40 includes a band rejection filter 410, an object lens 420, a wavelength separator 430, an infrared image detection unit 440, and a visible light image detection unit 450. The light detection device 40 identifies and detects light signals that are transferred to the light detection device 40 after pieces of light that are radiated from the endoscope 10 to the sample 60 are emitted from or reflected by the sample 60. Specifically, the light detection device 40 may detect at least one of a fluorescent signal that is emitted from the sample 60 and a visible light signals that is reflected by the sample 60, after light is radiated from the endoscope 10 to the sample 60. That is, the light detection device 40 may separate the fluorescent signal and the visible light signal through the wavelength separator 430, may identify and detect the fluorescent signal that is emitted from the sample 60 through the infrared image detection unit 440, and may identify and detect the visible light signal that is reflected by the sample 60 through the visible light detection unit 450. A detailed construction of the light detection device 40 is described later with reference to FIGS. 4 and 5.

The control device 50 synchronizes the endoscope 10 and the light detection device 40. The control device 50 may receive the data of the time when light is emitted from the endoscope 10 and the wavelength of the emitted light, and may receive the data of the time when light detected by the light detection device 40 is received and the wavelength of the detected light. That is, the control device 50 can secure information on the detected light more accurately by synchronizing the data of the time when the light is emitted from the endoscope 10 and the wavelength of the emitted light and the data of the time when the light is detected by the light detection device 40 and the wavelength of the detected light, and can identify and measure a multi-fluorescent signal.

The multi-spectrum endoscope system 1 according to an embodiment of the present disclosure may simultaneously radiate infrared rays and visible light having different wavelengths to the sample 60 through the endoscope 10 that is very small. The endoscope 10 emits a wavelength-variable light source, a multi-wavelength light source, a multi-array light source, etc., so that the multi-spectrum endoscope system 1 can obtain a fluorescent image having different wavelengths. A plurality of infrared rays and visible light that are radiated to the sample 60 as described above are received through the lens part 230 of the endoscope 10. The plurality of infrared rays and the visible light that have been received may be separated, identified, and detected through the light detection device 40. Accordingly, the multi-spectrum endoscope system 1 according to an embodiment of the present disclosure may identify different cells marked by different contrast media.

FIG. 2 is a cross-sectional view of the end part of the multi-spectrum endoscope toward a sample according to an embodiment of the present disclosure. That is, FIG. 2 is a cross-sectional view of the end part of the multi-spectrum endoscope, which is viewed in a direction II in FIG. 1. FIG. 3 is a cross-sectional view of the multi-spectrum endoscope according to an embodiment of the present disclosure, which is taken along in a direction III-III' in FIG. 1.

Referring to FIG. 2, the lens part 230 and the optical fiber bundle 240 that is connected to the lens part 230 are disposed at the center of the insertion unit 200 of the endoscope 10. The plurality of multi-mode optical fibers 210 is disposed in the periphery of the lens part 230 and the optical fiber bundle 240. The optical fiber or lighting part 220 that provides guidance for and emits visible light is disposed in the periphery of the plurality of multi-mode optical fibers 210. In FIG. 2, the lighting part 220 that is connected to the optical fibers and that has a doughnut shape has been illustrated in a form in which the lighting part 220 surrounds the plurality of multi-mode optical fibers 210. At least one optical fiber or lighting part 220 that provides guidance for and emits visible light may be constructed. Multiple optical fibers or lighting parts 220 may be disposed in the periphery of the outskirts of the plurality of multi-mode optical fibers 210. Alternatively, one or more optical fibers may be connected to one lighting part from which visible light is emitted. A structure or part that changes or reflects a light path so that visible light that is output from one or more optical fibers is properly emitted through the opening of the lighting part may be added. Accordingly, the visible light may be uniformly radiated toward the sample 60 through the one or more optical fibers or lighting parts 220.

Referring to FIG. 2, the plurality of multi-mode optical fibers 210 may emit infrared rays having different wavelengths λ₁, λ₂, λ₃, λ₄, λ₅, and λ₆, respectively, or may emit infrared rays having pieces of different predetermined power P₁, P₂, P₃, P₄, P₅, and P₆. According to various embodiments of the present disclosure, at least some of the plurality of multi-mode optical fibers 210 may output infrared rays having the same wavelength or the same power. For example, according to a user's selection or an embedded program, some multi-mode optical fibers 210a, 210c, and 210e may output infrared rays having the same wavelength or the same power, and the remaining multi-mode optical fibers 210b, 210d, and 210f may output infrared rays having wavelengths or power different from the wavelengths or power of the some multi-mode optical fibers 210a, 210c, and 210e. In FIG. 2, the plurality of multi-mode optical fibers 210 has been illustrated to have 6 strips, but the number of strips of the plurality of multi-mode optical fibers 210 is not limited thereto and may be an arbitrary number of 2 or more.

As described above, various infrared rays may be guided and emitted through the plurality of multi-mode optical fibers 210. In particular, such an infrared ray may be a near-infrared ray or a beam that is adjacent to the wavelength region of the near-infrared ray. Preferably, at least one of the plurality of multi-mode optical fibers 210 may output a beam between 200 nm to 450 nm, a beam between 510 nm to 550 nm, a near-infrared ray between 790 nm to 980 nm, or a near-infrared ray between 1044 nm to 1084 nm. For example, a near-infrared ray between 790 nm to 980 nm may be emitted through some multi-mode optical fibers 210a, 210c, and 210e. A near-infrared ray between 1044 nm to 1084 nm may be emitted or any light may not be emitted through the remaining multi-mode optical fibers 210b, 210d, and 210f.

Through the aforementioned embodiment, the multi-spectrum endoscope 10 according to an embodiment of the present disclosure may simultaneously emit an infrared ray corresponding to a contrast medium capable of identifying a cancer cell through the remaining multi-mode optical fibers 210b, 210d, and 210f while emitting an infrared ray capable of directly destructing the cancer cell through some multi-mode optical fibers 210a, 210c, and 210e. Accordingly, the multi-spectrum endoscope 10 may emit an infrared ray having different wavelengths or power corresponding to a plurality of contrast media so that a plurality of cancer cells and other tissues can be identified, and may simultaneously handle a cancer cell directly.

Furthermore, the optical fiber or lighting part 220 is disposed to surround the plurality of multi-mode optical fibers 210, and provides guidance for and outputs visible light outside ultraviolet rays. Light that is emitted through the optical fiber or lighting part 220 may be white light. Such white light may be implemented by using a white LED. A visible light that is output through the optical fiber or lighting part 220 plays a role as illumination light so that a field of view can be secured when the multi-spectrum endoscope 10 is inserted into the human body, the body of an animal, or a dark structure, for example. As infrared rays having a plurality of wavelengths and visible light are simultaneously emitted toward the sample 60 through one multi-spectrum endoscope 10, visible light that is emitted through the optical fiber or lighting part 220 plays a role as illumination light, and enable a bright and sharp image to be obtained.

Referring to FIG. 2, the lens part 230 may be disposed at the center of the insertion unit 200 of the multi-spectrum endoscope 10. The lens part 230 may be disposed to be surrounded by the plurality of multi-mode optical fibers 210. The lens part 230 receives a fluorescent signal that is emitted from the sample 60 and a visible light signal that is reflected by the sample. The lens part 230 is connected to the optical fiber bundle for transferring the received fluorescent signal and visible light signal to the light detection device. A relationship between the lens part 230 and the optical fiber bundle 240 is described later with reference to FIG. 3.

Referring to FIG. 3, the optical fiber bundle 240 is connected to the rear of the lens part 230, so that light signals received through the lens part 230 are guided to the light detection device through the optical fiber bundle 240. The optical fiber bundle 240 is a bundle in which a plurality of optical fibers gathers. The plurality of optical fibers may be mutually twisted and united in a screw form. The plurality of multi-mode optical fibers 210 may be disposed to surround the optical fiber bundle 240 in the periphery of the outside of the optical fiber bundle 240. The optical fiber or lighting part 220 may be disposed to surround the plurality of multi-mode optical fibers 210. In FIG. 3, the plurality of multi-mode optical fibers 210 has been illustrated as being disposed in a straight line. However, the plurality of multi-mode optical fibers 210 according to various embodiments may also be disposed to be twisted in a screw form while surrounding the outside of the optical fiber bundle 240.

Furthermore, the lens part 230 and a homogenizer or diffuser 215 may be disposed at an end part of the insertion unit 200. An end part of each of the plurality of multi-mode optical fibers 210 from which a first light source is emitted includes the homogenizer or diffuser 215. The homogenizer or diffuser 215 enables infrared rays that are output from the plurality of multi-mode optical fibers 210 to be uniformly diffused. Accordingly, as the infrared rays emitted through the homogenizer or diffuser 215 are uniformly diffused, speckle noise and multi-modal noise between the infrared rays can be removed or reduced. Accordingly, the multi-spectrum endoscope 10 according to an embodiment of the present disclosure removes speckle noise and multi-modal noise while emitting a plurality of infrared rays and visible light so that a clearer multi-spectrum fluorescent image can be obtained.

FIG. 4 is a schematic block diagram of a multi-spectrum light detection device that is connected to the multi-spectrum endoscope according to an embodiment of the present disclosure.

Referring to FIGS. 1 and 4, light that is emitted from the multi-spectrum endoscope 10 reaches the sample 60. A light signal that has reached the light detection device 40 is separated into a visible light signal that is reflected by the sample 60 and an infrared ray signal that is emitted from the sample 60, so that the light detection device 40 may separately identify and detect the visible light signal and the infrared ray signal. In this case, the infrared ray signal is a fluorescent signal. The light detection device 40 includes the band rejection filter (BRF) 410, the object lens 420, the wavelength separator 430, the infrared image detection unit 440, and the visible light image detection unit 450.

Referring to FIG. 4, the band rejection filter 410 is disposed between the sample 60 and the wavelength separator 430. Furthermore, the band rejection filter 410 may be disposed close to the sample 60 than to the object lens 420. The band rejection filter 410 may remove the component of exciting light from a light signal by removing the wavelength of the exciting light from a light signal that is reflected or discharged from the sample 60. Accordingly, the light detection device 40 may receive a near-infrared ray signal according to pieces of light that is emitted from the light source 110 and that corresponds to a specific wavelength and a visible light signal according to illumination light, without noise according to the exciting light. In this case, as a result, the near-infrared ray signal corresponds to the fluorescent signal, and thus the remaining near-infrared ray signals except the visible light signal may be used as the same meaning as the fluorescent signal.

The light signal from which the component of the exciting light has been removed by the band rejection filter 410 reaches the wavelength separator 430 through the object lens 420 adjacent to the band rejection filter 410. The wavelength separator 430 is a component that separates light signals on the basis of a desired wavelength, and may be a dichroic mirror or a beam splitter, for example.

The fluorescent signal includes a component of an infrared ray that is emitted from the first light source 110, which corresponds to a specific wavelength. The visible light signal includes a component of illumination light that is visible light emitted from the second light source 120, which is reflected by the sample 60. Accordingly, the wavelength separator 430 separates the light signal into a visible light signal that is reflected by the sample 60 and a fluorescent signal that is emitted from the sample 60. The fluorescent signal that is separated by the wavelength separator 430 is transmitted to the infrared image detection unit 440. The visible light signal that is separated by the wavelength separator 430 is transmitted to the visible light image detection unit 450.

Referring to FIG. 4, the infrared image detection unit 440 may further include a wheel filter 443 or 445, an infrared object lens 441, and an infrared image sensor 442. Accordingly, the infrared image detection unit 440 detects fluorescent signals corresponding to a plurality of wavelengths, which are separated by the wavelength separator 430, through the infrared image sensor 442.

Furthermore, the visible light image detection unit 450 may include a visible light object lens 451 and a visible light image sensor 450. Accordingly, the visible light image detection unit 450 detects a visible light signal that is separated by the wavelength separator 430 through the visible light image sensor 450.

According to another embodiment of the present disclosure, the light detection device 40 may further include the wheel filter 443 or 445. The wheel filter 443 or 445 is a filter having a wheel form, and may include a plurality of bandpass filters (BPFs). Preferably, the bandpass filters may be disposed along the circumstance of the wheel filter 443 or 445 at predetermined intervals. Each of the bandpass filters may transmit only light signal having a specific wavelength component in the fluorescent signal that is emitted from the sample 60.

For example, the wheel filter 443 or 445 may include n bandpass filters. Each of the bandpass filter may transmit a near-infrared light having n different specific wavelength ranges. Accordingly, although light that is incident on the wheel filter 443 or 445 has n or more wavelength types, a light signal that passes through the wheel filter 443 or 445 may include only a signal component within a specific wavelength range.

In FIG. 4, the wheel filter 445 has been illustrated as being disposed between the wavelength separator 430 and object lens 420, but this is merely an example according to one of various embodiments. The wheel filter 445 may be disposed at the place where a wavelength will be made to selectively pass. Specifically, in an embodiment, the wheel filter 443 may be disposed between the wavelength separator 430 and the infrared image sensor 442. In another embodiment, the wheel filter 445 may be disposed between the wavelength separator 430 and the band rejection filter 410. More specifically, as in FIG. 4, the wheel filter 443 may be disposed between the wavelength separator 430 and the infrared object lens 441 that is disposed ahead of the infrared image sensor 442. The wheel filter 445 may be disposed between the wavelength separator 430 and the object lens 420 that is disposed behind the band rejection filter 410.

The fluorescent signal that is separated by the wavelength separator 430 reaches the infrared image sensor 442 via the infrared object lens 441. The infrared image sensor 442 may detect a near-infrared ray signal having a plurality of wavelengths. In particular, the infrared image sensor 442 may detect fluorescent signals having a plurality of wavelengths by separating the fluorescent signals into fluorescent signals for each wavelength. Accordingly, the infrared image sensor 442 transmits a detected fluorescent signal for each wavelength to the control device 50, etc. so that a near-infrared fluorescent image that has been divided for each wavelength can be output.

Specifically, the infrared image sensor 442 may receive the data of the time and wavelength of an infrared ray that is emitted from the first light source 110, and may identify and detect a multi-wavelength fluorescent signal in synchronization with the data. Furthermore, the infrared image sensor 442 may also be synchronized with the wheel filter 443 or 445. The wheel filter 443 or 445 may also identify and detect a multi-wavelength fluorescent signal in synchronization with the first light source 110. A detailed method of the infrared image sensor 442 being synchronized with the first light source 110 and the wheel filter 443 or 445 is described later with reference to FIG. 5.

The visible light signal that is separated by the wavelength separator 430 reaches the visible light image sensor 452 via the visible light object lens 451. The visible light image sensor 452 may detect the visible light signal. Accordingly, the visible light image sensor 452 transmits the detected visible light signal to the control device 50, etc. so that a visible light image is output.

As described above, pieces of light that are emitted from the multi-spectrum endoscope 10 according to an embodiment of the present disclosure toward the sample 60 are emitted or reflected by the sample 60, and reach the light detection device 40 through the lens part 230. Accordingly, various light signals that have reached the light detection device 40 include a fluorescent signal having a plurality of wavelengths and a visible light signal. The light detection device 40 according to an embodiment of the present disclosure may detect a plurality of fluorescent signals corresponding to a plurality of wavelengths, respectively, and a visible light signal by separating the plurality of fluorescent signals and the visible light signal through the wavelength separator 430.

The fluorescent signal corresponding to the plurality of wavelengths corresponds to a fluorescent signal corresponding to an infrared ray having a plurality of wavelengths, which is emitted from the first light source 110. The visible light signal corresponds to a signal corresponding to visible light that is emitted from the second light source 120. Accordingly, the light detection device 40 may detect the fluorescent signals corresponding to the plurality of wavelengths, respectively, and the visible light signals by separating the fluorescent signals and the visible light signal through the wavelength separator 430. That is, in one multi-spectrum endoscope system 1 according to an embodiment of the present disclosure, the light detection device 40 may simultaneously receive and detect the fluorescent signals having the plurality of wavelengths and the visible light signal, may separate the plurality of fluorescent signals and the visible light signal according to their wavelength, and may separately indicate the plurality of fluorescent signals and the visible light signal that have been separated as described above by predetermined specific colors.

As a result, the multi-spectrum endoscope system 1 including the light detection device 40 according to an embodiment of the present disclosure may emit light having a plurality of wavelengths to the sample 60 at a time by using a multi-wavelength light source, a multi-array light source, or a wavelength-variable light source through the multi-spectrum endoscope 10, may separate a visible light signal and fluorescent signals for each wavelength from light signals having a plurality of wavelengths, which are emitted or reflected by the sample 60, at a time, and may output the visible light signal and the fluorescent signals with different colors by dividing the visible light signal and the fluorescent signals.

FIG. 5 is a block diagram for describing a method of identifying and measuring a multi-wavelength fluorescent signal by synchronizing the multi-spectrum endoscope and the multi-spectrum light detection device in the endoscope system according to an embodiment of the present disclosure. FIG. 5 has substantially the same components as FIG. 4 and is for describing a synchronizing method, and a redundant description of the component is omitted.

Referring to FIG. 5, information on light that is emitted and transferred between a light source 100 and the infrared image sensor 442 is synchronized (510). Information on light that is emitted and transferred between the light source 110 and the wheel filter 443 or 445 is synchronized (520). Information on light that is emitted and transferred between the wheel filter 443 or 445 and the infrared image sensor 442 is synchronized (530).

Such a synchronization process may be performed through the control device 50 that is connected to the multi-spectrum endoscope 10 and the light detection device 40. Specifically, the control device 50 may synchronize a time between the first light source 110 of the light source 100 and the infrared image sensor 442 and the wavelength of light corresponding to the time (510), may synchronize a time between the first light source 110 and the wheel filter 443 or 445 and the wavelength of light corresponding to the time (520), and may synchronize a time between the wheel filter 443 or 445 and the infrared image sensor 442 and the wavelength of light corresponding to the time (530). More specifically, the control device 50 may receive or store information on the wavelength of an infrared ray that is emitted from the first light source 110 and the time when the infrared ray having the corresponding wavelength is emitted, and may transmit the information on the wavelength of the emitted infrared ray and the time when the corresponding infrared ray is emitted to the infrared image sensor 442 and the wheel filter 443 or 445. Accordingly, the infrared image sensor 442 and the wheel filter 443 or 445 may receive information on when an infrared ray having a specific wavelength is emitted from the first light source 110. Furthermore, the wheel filter 443 or 445 and the infrared image sensor 442 may detect a fluorescent signal having a specific wavelength more accurately and identify and measure a fluorescent signal having multiple wavelengths, by detecting the fluorescent signal having the specific wavelength from a fluorescent signal that is emitted from the sample 60 and synchronizing the time when the corresponding fluorescent signal having the specific wavelength is emit.

The multi-spectrum endoscope system 1 according to an embodiment of the present disclosure may synchronize information on times and wavelengths for pieces of light that are emitted from the multi-spectrum endoscope 10 and information on times and wavelengths for pieces of light that are received by the light detection device 40. Accordingly, the light detection device 40 of the multi-spectrum endoscope system 1 can separate, identify, and detect individual fluorescent signals having a plurality of wavelengths more accurately on the basis of the synchronized information on the light. Accordingly, if the multi-spectrum endoscope system 1 is used, although pieces of light having a plurality of wavelengths are emitted at a time or with some time difference, various types of contrast media can be used at a time because a fluorescent signal for each wavelength can be detected and identified accurately. Furthermore, lesions at several parts or the state of a cell can be checked at a time because the lesions or the state of the cell can be distinguished at a time by using several fluorescent signals having different wavelengths according to various types of contrast media.

In this specification, each block may represent a portion of a module, a segment, or a code, which includes one or more executable instructions for executing a specified logical function(s). Furthermore, it should also be noted that in some alternative embodiments, the functions noted in the blocks may occur out of order. For example, two blocks shown in succession may in fact be executed substantially concurrently, or the blocks or steps may sometimes be executed in the reverse order, depending on their functions involved.

The embodiments of the present disclosure have been described more specifically with reference to the accompanying drawings, but the present disclosure is not essentially limited to such embodiments and may be modified and implemented in various ways without departing from the technical spirit of the present disclosure. Accordingly, the embodiments disclosed in the present disclosure should not be construed as limiting the technical spirit of the present disclosure, but should be construed as describing the technical spirit of the present disclosure. Accordingly, it should be construed that the aforementioned embodiments are only illustrative in all aspects, and are not limitative. The range of protection of the present disclosure should be construed based on the following claims, and all of technical spirits within an equivalent range of the present disclosure should be construed as being included in the scope of rights of the present disclosure.

## Claims

1. A multi-spectrum endoscope comprising:
a first light source configured to emit an infrared ray having a plurality of wavelengths;
a plurality of multi-mode optical fibers configured to provide an infrared ray that is emitted from the first light source with guidance toward a sample;
a second light source configured to emit visible light;
an optical fiber configured to provide the infrared ray that is emitted from the second light source with guidance toward the sample;
a lens part configured to receive a fluorescent signal that is emitted from the sample and a visible light signal that is reflected by the sample; and
an optical fiber bundle configured to provide at least one of the fluorescent signal and the visible light signal that are received through the lens part with guidance toward a multi-spectrum light detection device,
wherein the optical fiber bundle is disposed to be surrounded by the plurality of multi-mode optical fibers, and an end part of the multi-spectrum endoscope from which the visible light is emitted surrounds at least some of the plurality of multi-mode optical fibers.

2. The multi-spectrum endoscope of claim 1, further comprising a coupler configured to separate the infrared ray having the plurality of wavelengths into individual infrared rays having different wavelengths or having pieces of different predetermined power.

3. The multi-spectrum endoscope of claim 2, wherein the coupler transmits at least one of the fluorescent signal and the visible light signal toward the light detection device.

4. The multi-spectrum endoscope of claim 2, wherein each of the plurality of multi-mode optical fibers outputs the individual infrared ray separated by the coupler by providing the individual infrared ray with guidance toward the sample.

5. The multi-spectrum endoscope of claim 1, wherein the first light source is a wavelength-variable light source, a multi-wavelength light source, or a multi-array light source, and emits light having a different wavelength over time.

6. The multi-spectrum endoscope of claim 1, wherein at least one of the plurality of multi-mode optical fibers outputs a beam between 200 nm to 450 nm, a beam between 510 nm to 550 nm, a near-infrared ray between 790 nm to 980 nm, or a near-infrared ray between 1044 nm to 1084 nm.

7. The multi-spectrum endoscope of claim 1, wherein an end part of each of the plurality of multi-mode optical fibers from which the first light source is emitted comprises a homogenizer or a diffuser.

8. An endoscope system comprising:
a multi-spectrum endoscope according to claim 1;
a multi-spectrum light detection device configured to detect at least one of a fluorescent signal that is radiated from the multi-spectrum endoscope to a sample and that is then emitted from the sample and a visible light signal that is reflected by the sample; and
a control device configured to synchronize the multi-spectrum endoscope and the multi-spectrum light detection device.

9. The endoscope system of claim 8, wherein the multi-spectrum light detection device comprises:
a wavelength separator configured to separate the fluorescent signal and the visible light signal;
a band rejection filter disposed between the sample and the wavelength separator;
an infrared image detection unit configured to detect the fluorescent signal; and
a visible light image detection unit configured to detect the visible light signal.

10. The endoscope system of claim 9, wherein:
the infrared image detection unit comprises an infrared image sensor, an infrared object lens, and a wheel filter,
the visible light image detection unit comprises a visible light image sensor and a visible light object lens, and
the wheel filter is disposed between the wavelength separator and the infrared image sensor or between the wavelength separator and the band rejection filter.

11. The endoscope system of claim 10, wherein the control device synchronizes a time between the first light source and the infrared image sensor and a wavelength of light corresponding to the time, synchronizes a time between the first light source and the wheel filter and a wavelength of light corresponding to the time, and synchronizes a time between the wheel filter and the infrared image sensor and a wavelength of light corresponding to the time.
